# EUROPÄISCHE PATENTSCHRIFT

(11) **EP 0 820 316 B1**
(45) Veröffentlichungstag und Bekanntmachung des Hinweises auf die Patenterteilung: **12.06.2002**
(21) Anmeldenummer: 96903986.6
(22) Anmeldetag: 06.02.1996
(51) Int. Cl.: A61L 33/00

(54) **BESCHICHTUNG FÜR IN DEN BLUTSTROM ODER IN DAS GEWEBE DES MENSCHLICHEN KÖRPERS EINBRINGBARES BIOMATERIAL**
COATING FOR BIO-MATERIAL INSERTABLE INTO THE BLOODSTREAM OR TISSUE OF THE HUMAN BODY
REVETEMENT DESTINE A UNE MATIERE BIOLOGIQUE POUVANT ETRE INTRODUITE DANS LE FLUX SANGUIN OU DANS LE TISSU DU CORPS HUMAIN

(30) Priorität: 13.04.1995 DE 19514104
(43) Veröffentlichungstag der Anmeldung: 28.01.1998
(73) Patentinhaber: Stemberger, Axel, Dr., D-85579 Neubiberg (DE); Alt, Eckhard, Dr., D-85521 Ottobrunn (DE)
(72) Erfinder: REERS, Martin, 35043 Marburg (DE); STÜBER, Werner, 35094 Lahntal (DE); STEMBERGER, Axel, 85579 Neubiberg (DE); ALT, Eckhard, 85521 Ottobrunn (DE)
(74) Vertreter: Haft, von Puttkamer, Berngruber, Czybulka
(86) Internationale Anmeldenummer: EP9600471
(87) Internationale Veröffentlichungsnummer: WO9632143

(56) Entgegenhaltungen:
- EP-A- 0 299 698
- EP-A- 0 442 843
- EP-A- 0 513 543
- WO-A-94/26399
- DE-A- 4 334 272

## Beschreibung

Die Erfindung bezieht sich auf eine Beschichtung für in den Blutstrom oder in das Gewebe des menschlichen Körpers einbringbares Biomaterial. Mit diesem Ausdruck werden z.B. Infusionskatheter, Herzkatheter, Ballonkatheter, Elektroden, Nahtmaterial für Gefäßanastomosen, Oxygenatoren, Gefäßprothesen oder Stützkörper für Gefäße, sogenannte Stents, etc. bezeichnet, die kurzfristig aber auch längerfristig unmittelbar in Arterien und Venen sowie in Körpergewebe verbracht werden oder mit Blut in Kontakt kommen. Die Gefährdungen der Patienten, z.B. durch Thrombosenbildung und Entzündungen, sind bekannt und hinsichtlich Therapieerfolg mit dem Schweregrad der Erkrankung abzuwägen.

Es ist aus der EP-A1-0578998 bekannt, derartiges Biomaterial mit einer Umkleidung aus einem biologisch abbaubaren Material, z.B. Poly-D,L-Laktid auszubilden, wobei dann in dieses biologisch abbaubare Material noch Medikamente eingearbeitet sind, die beim Abbau des Biomateriales im implantierten Zustand nach und nach mit vorzugsweise gleichbleibender Rate an den Patienten abgegeben werden. Als Medikament wird z.B. Heparin erwähnt, das, in dispergierter Form eingearbeitet, dann gezielt in den Blutkreislauf gelangt und als Katalysator plasmaständiger Inhibitoren wie Antithrombin III bzw. Heparin-Cofaktor II deren Wirkung beschleunigt.

Es ist ferner in den deutschen Patentanmeldungen P 43 34 272.8 sowie P 44 35 652.8 vorgeschlagen worden, Biomaterial mit einem biologisch abbaubaren Material zu beschichten, wobei die Beschichtung sehr dünn mit Schichtdicken kleiner als 100 Mikrometer ist, so daß bei dem Biomaterial lediglich die Primärstruktur bedeckt wird. Ist z.B. die Primärstruktur eine Netzstruktur, wie dieses bei den genannten Gefäßprothesen oder Stents der Fall ist, so werden lediglich die einzelnen Stränge der Prothese beschichtet; es wird in keinem Fall die Prothese mit einer vollständigen Hülle umgeben. Bei einer solchen lackartigen Beschichtung hat sich gezeigt, daß durch den langsamen biologischen Mikroabbau des Beschichtungsmaterials bereits ein antithrombogener Effekt erzielt wird. Als Beschichtungsmaterial werden hierbeibiodegradierbare synthetische Polymere, wie Polyglykole und Polylaktide sowie entsprechende Mischpolymerisate oder Mischungen etc. verwendet, die in einem organischen Lösungsmittel, vorzugsweise Chloroform gelöst sind, welches nach Auftragen auf das Biomaterial verdampft.

In diesen Patentanmeldungen ist auch vorgeschlagen, in das Beschichtungsmaterial Arzneistoffe einzuarbeiten, wobei als Arzneistoffe sowohl die Blutgerinnung hemmende als auch entzündungshemmende Arzneistoffe verwendet werden. Auch das Einarbeiten von Antibiotika ist möglich. Im Gegensatz zu dem vorerwähnten Verfahren sollen diese Arzneistoffe jedoch nicht in hohem Maße in den Blutstrom gegeben werden, sondern im wesentlichen lokal wirken.

In Versuchen hat sich herausgestellt, daß mit einer solchen lackartigen Beschichtung, ggf. in Kombination mit eingearbeiteten Arzneistoffen die Ausbildung von Thromben im weiten Umfang verhindert werden kann, so daß die ansonsten immer zu befürchtenden Operationsrisiken stark reduziert werden können.

Der Erfindung liegt die Aufgabe zugrunde, ein Beschichtungsmaterial anzugeben, bei dem die vorteilhaften Wirkungen weiter gesteigert werden können.

Diese Aufgabe ist gemäß der Erfindung durch die Merkmale des Patentanspruches 1 gelöst.

Demnach wird zumindest ein Inhibitor gegen Serinproteasen verwendet, der ebenso wie der Arzneistoffträger selbst, in dem zur Präparation des Beschichtungsmaterials notwendigen organischen Lösungsmittel, vorzugsweise Chloroform, selbst lösbar ist. Hiermit ergibt sich eine homogene Lösung, die auf das Biomaterial aufgebracht wird, wonach anschließend das Lösungsmittel verdampft, so daß dann ein gelöstes homogenes Gemenge auf dem Biomaterial als Beschichtung vorliegt. Der Inhibitor ist ein direkt wirkender Thrombininhibitor, d.h. wirkt ohne Zwischenschaltung eines körpereigenen Cofaktors oder dgl..

Als weiteres homogen gelöstes Medikament kann noch ein Prostaglandin bzw. Prostacyclin vorliegen, wobei eine zusätzliche Einarbeitung von schnell wirkenden Antithrombogenen, wie Hirudin, möglich ist.

Als Arzneistoffträger dient bevorzugt ein Poly-D,L-Laktid, welches als R203 der Firma Boehringer, Ingelheim, käuflich ist, verwendet; als Antithrombin findet bevorzugt ein Amindinophenylanalinderivat gemäß den Ansprüchen 8 und 9 Verwendung, das von den Behring-Werken AG unter der Bezeichnung CRC220 vertrieben wird und näher in der EP-A1-0513543 beschrieben ist.

Das dort beschriebene lösliche Antithrombin ist eine Verbindung der folgenden Struktur: 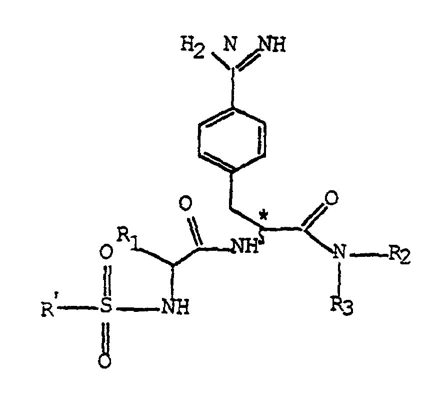 worin
R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist,
   oder ein Phenylring ist, der gegebenenfalls mit Alkylgruppen, die bis zu 4 C-Atome enthalten, und/oder mit bis zu drei Gruppen der Struktur O-X, in der O Sauerstoff und X Wasserstoff, Methyl, Ethyl, n-Propyl, i-Propyl oder tert.-Butyl ist, und/oder mit einer Gruppe der Struktur -COOY, in der Y Wasserstoff, Methyl, Ethyl, n-Propyl, i-Propyl, tert.-Butyl, 1-Butyl, i-Pentyl oder neo-Pentyl ist, derivatisiert ist,
   oder ein Chromansystem ist, das vorzugsweise mit bis zu 5 Alkylgruppen, die bis zu 3 C-Atome enthalten, derivatisiert ist,
R1 eine Gruppe der Struktur A-B ist, wobei A = -(CH₂)ₙ- und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17 C-Atomen enthalten, Sulfonsäurefunktion, eine Funktion einer Säure des Phosphors, eine Boronsäurefunktion und Tetrazolgruppe, oder R₁ eine Gruppe der Struktur A-B-C ist, wobei A obige Bedeutung hat, B Carbonyl oder Sulfonyl ist und die Gruppe C sich von einer N-gebundenen alpha, beta, gamma oder delta Aminosäure oder der Gruppe der N-glycosidisch verknüpften Uronsäuren ableitet, und
R₂ und R₃ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkylgruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in der das mit * gezeichnete C-Atom in der R oder S Struktur, vorzugsweise aber in der R-Struktur vorliegt.
R' ist hierbei bevorzugt 4-Methoxy-2,3,6-trimethylphenyl-, R₁ -CH₂-COOX mit X gleich Wasserstoff und R₂ und R₃ zusammen Piperidin.

Die Komponenten der Beschichtung liegen bevorzugt in einem Lösungsmittel, vorzugsweise Chloroform, gelöst vor, wobei die Anteile der einzelnen Komponenten pro Milliliter des Lösungsmittels folgende Werte betragen:
100 bis 300, vorzugsweise 150 bis 160 Milligramm Arzneistoffträger;
0,5 bis 20 Gew.-%, vorzugsweise bis 10 Gew.-% eines direkt wirkenden Antithrombins (CRC220) ;
0,3 bis 2 Gew.-%, vorzugsweise 0,5 - 1 Gew.-% eines Prostaglandinderivats oder eines entsprechenden Analogens;
0,5 bis 10 % Hirudin.

Die Beschichtung weist bevorzugt 5 Gew.-% jeder einzelnen Substanz auf..

Die Beschichtung bildet auf dem Biomaterial eine lackartige Haftschicht mit Schichtdicken kleiner als 100 Mikrometern, vorzugsweise kleiner als 50 Mikrometern bzw. 10 Mikrometern bildet.

Mit der Vorgabe eines Beschichtungsmateriales gemäß der vorliegenden Erfindung wurde Von dem Gedanken ausgegangen, daß Endothelzellen als innere Auskleidungen von Blutgefäßen Mechanismen besitzen, die ein Anhaften von Zellen und plasmatischen Proteinen verhindern. Hierbei verhindern insbesondere freigesetzte Substanzen wie Prostaglandine die Anlagerung von Blutplättchen; weiterhin werden an der Oberfläche Substanzen produziert, die einer Thrombinbildurig entgegenwirken.

Im Rahmen der Versuche zur vorliegenden Erfindung hat sich nun gezeigt, daß sogenannte selbstreinigende Oberflächen, d.h. permanent biologisch abbaubare Materialien, in Kombination mit homogen verteilten Thrombin- bzw. Serinproteasen-Inhibitoren sowie gegebenenfalls Prostaglandinen oder Prostazyklinderivaten bzw. entsprechenden Analoga, die in gelöster Form in die Beschichtungsmaterialien eingebracht sind, eine endothel-ähnliche Wirkung besitzen. Hierdurch wird bei nur geringer systemischer Verfügbarkeit der eingearbeiteten gelösten Medikamente durch Kombination mit der selbstreinigenden Oberflächenbeschichtung die Anlagerung, die Aktivierung der plasmatischen Gerinnung und die Aggregation von Blutplättchen verhindert. Das Beschichtungsmaterial kann sozusagen als Langzeitdepot bezeichnet werden, wobei es Ziel ist, die Freigabe der Medikamente, insbesondere der Antithrombine möglichst gering zu halten, um nicht die bekannten Nachteile systemischer Dosierung zu erhalten.

Beim Einbringen von Implantaten oder Prothesen in den Blutkreislauf, die mit einer besonders hohen Gefahr der Aktivierung der Gerinnung verbunden sind, hat sich eine sogenannte 3er Kombination bestehend aus Thrombininhibitoren wie Hirudin und dem genannten CRC220 mit einem synthetischen Prostaglandinderivat (Iloprost) bewährt. Hierbei dient das Hirudin als unmittelbar in der operativen Phase schnell verfügbarer Thrombininhibitor, durch den die Komplikation des operativen Eingriffs reduziert wird. Die weiterhin in dem Arzneistoffträger homogen verteilten Inhibitoren bewirken dann die notwendige Langzeitkompatibilität.

Zur Herstellung des Beschichtungsmaterials gemäß der Erfindung wird zunächst eine Grundlösung hergestellt aus einem Arzneistoffträger, vorzugsweise Poly-D,L-Laktid und einem Lösungsmittel, vorzugsweise Chloroform. Für die Lösung werden in einem Milliliter Lösungsmittel, vorzugsweise Chloroform, 50 bis 300 Milligramm, vorzugsweise 150 bis 160 Milligramm eines Arzneistoffträgers gelöst.

In dieser Grundlösung wird das erwähnte Antithrombin CRC220 gelöst, so daß sich in der Endmischung in bezug auf den Arzneistoffträger ein Gehalt zwischen 0,5 bis ca. 20 Gew.-% ergibt, bevorzugt bis 10 Gew.-%. Des weiteren wird noch das erwähnte Prostaglandinderivat Iloprost in einem Anteil zwischen 0,5 und 7 Gew.-%, vorzugsweise 1 bis 5 Gew.-% gelöst und schließlich Hirudin in einem Anteil zwischen 2 und 10 Gew.-% der Gesamtlösung, vorzugsweise etwa 5 Gew.-% zugesetzt.

Nach Auftragen dieser Lösung auf Biomaterial verdunstet das Chloroform, so daß dann ein homogenes Gemenge aus Arzneistoffträger und zugesetzten Medikamenten als Beschichtung vorliegt.

## Patentansprüche

1. Beschichtung für in den Blutstrom oder in das Gewebe des menschlichen Körpers einbringbares Biomaterial, wie Infusions-, Herz- oder Ballonkatheter, Elektroden für Herzschrittmacher und Defibrillatoren, Nahtmaterial, Oxygenatoren, Stützkonstruktionen für Gefäße (Stents) oder dergleichen, wobei durch diese Beschichtung insbesondere die Blutgerinnung an dem Biomaterial durch Anhaften von plasmatischen oder zellulären Bestandteilen verhindert wird und die Beschichtung einen blut- und gewebeverträglichen Arzneistoffträger aufweist, der in einem organischen Lösungsmittel gelöst wird, in den wenigstens ein Medikament eingelagert ist und der nach Auftragen auf das Biomaterial und Verdampfen des Lösungsmittels im Körper permanent biologisch abgebaut wird, **dadurch gekennzeichnet, daß** in dem Arzneistoffträger ein Inhibitor gegen Serinproteasen in homogen verteilter gelöster Form vorliegt, wobei dieser Inhibitor gemeinsam mit dem Arzneistoffträger in dem gleichen organischen Lösungsmittel lösbar ist, so daß sich nach Auftragen auf das Biomaterial und Verdampfen des Lösungsmittels eine homogene Beschichtung aus Arzneistoffträger und Inhibitor ergibt, die eine dem Endothel vergleichbare Funktion aufweist.

2. Beschichtung nach Anspruch 1, **dadurch gekennzeichnet, daß** der Inhibitor ein direkt wirkender Thrombininhibitor ist.

3. Beschichtung nach Anspruch 2, **dadurch gekennzeichnet, daß** der Thrombininhibitor die Kontaktaktivierung der Blutgerinnung unterbindet.

4. Beschichtung nach einem der vorhergehenden Ansprüche, **dadurch gekennzeichnet, daß** zur Erzielung einer endothelähnlichen Wirkung des weiteren ein Prostaglandin bzw. Prostacyclin oder ein entsprechendes Derivat gemeinsam mit dem Arzneistoffträger und dem Inhibitor dem Lösungsmittel zugegeben wird und in gelöster Form in dem Arzneistoffträger enthalten ist.

5. Beschichtung nach einem der vorhergehenden Ansprüche, **dadurch gekennzeichnet, daß** in den Arzneistoffträger zusätzlich ein rasch freigebbares Antithrombin, wie Hirudin, eingearbeitet ist.

6. Beschichtung nach einem der vorhergehenden Ansprüche, **dadurch gekennzeichnet, daß** als Arzneistoffträger ein Poly-D,L-Laktid verwendet wird.

7. Beschichtung nach einem der vorhergehenden Ansprüche, **dadurch gekennzeichnet, daß** die einzelnen Komponenten der Beschichtung in Chloroform gelöst sind.

8. Beschichtung nach einem der vorhergehenden Ansprüche, **dadurch gekennzeichnet, daß** die Beschichtung auf dem Biomaterial eine lackartige Haftschicht mit Schichtdicken kleiner als 100 Mikrometern, vorzugsweise kleiner als 50 Mikrometern bzw. 10 Mikrometern bildet.

9. Beschichtung nach einem der vorhergehenden Ansprüche, **dadurch gekennzeichnet, daß** als lösliches Antithrombin eine Verbindung der folgenden Struktur verwendet wird: 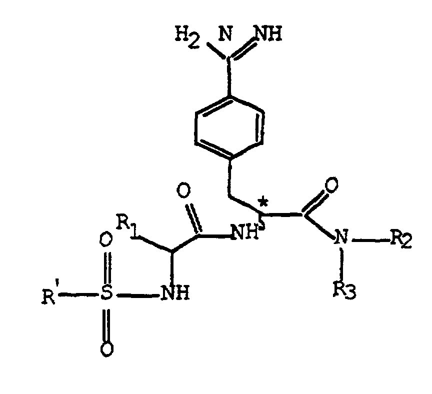 worin
R' ein in der alpha- oder beta-Position gebundener Naphthalinring ist, der gegebenenfalls mit Alkylgruppen, die bis zu 3 C-Atome enthalten, und/oder Alkoxygruppen mit jeweils bis zu 3 C-Atomen, derivatisiert ist, oder ein in der alpha- oder beta-Position gebundener Tetralinring oder Indanring ist, der gegebenenfalls mit Alkylgruppen, die aus bis zu 3 C-Atomen bestehen, und/oder auch Alkoxygruppen mit jeweils bis zu 3 C-Atomen derivatisiert ist,
oder ein Phenylring ist, der gegebenenfalls mit Alkylgruppen, die bis zu 4 C-Atome enthalten, und/oder mit bis zu drei Gruppen der Struktur O-X, in der O Sauerstoff und X Wasserstoff, Methyl, Ethyl, n-Propyl, i-Propyl oder tert..-Butyl ist, und/oder mit einer Gruppe der Struktur -COOY, in der Y Wasserstoff, Methyl, Ethyl, n-Propyl, i-Propyl, tert.-Butyl, 1-Butyl, i-Pentyl oder neo-Pentyl ist, derivatisiert ist,
oder ein Chromansystem ist, das vorzugsweise mit bis zu 5 Alkylgruppen, die bis zu 3 C-Atome enthalten, derivatisiert ist,
R₁ eine Gruppe der Struktur A-B ist, wobei A = -(CH₂)ₙ- und n = 1-4 und B eine Säurefunktion ist, ausgewählt aus der Gruppe Carboxylfunktion, die gegebenenfalls verestert sein kann oder als Amid vorliegt, wobei die Ester einen Alkohol mit bis zu 17 C-Atomen enthalten, Sulfonsäurefunktion, eine Funktion einer Säure des Phosphors, eine Boronsäurefunktion und Tetrazolgruppe, oder R₁ eine Gruppe der Struktur A-B-C ist, wobei A obige Bedeutung hat, B Carbonyl oder Sulfonyl ist und die Gruppe C sich von einer N-gebundenen alpha, beta, gamma oder delta Aminosäure oder der Gruppe der N-glycosidisch verknüpften Uronsäuren ableitet, und
R₂ und R₃ gleich oder verschieden sein können und Alkylgruppen mit bis zu 4 C-Atomen sind oder zusammen einen heterocyclischen Ring mit bis zu 8 Ringgliedern bilden, der mit einer Hydroxygruppe oder einer Hydroxyalkylgruppe mit bis zu 3 C-Atomen derivatisiert sein kann und diese Hydroxygruppe gegebenenfalls verestert vorliegt, wobei die entsprechenden Säuren Carbonsäuren sind, die bis zu 17 C-Atome enthalten, und in der das mit * gezeichnete C-Atom in der R oder S Struktur, vorzugsweise aber in der R-Struktur vorliegt (CRC220).

10. Beschichtung nach Anspruch 9, **dadurch gekennzeichnet, daß** R' 4-Methoxy-2,3,6-trimethylphenyl-, R₁ -CH₂-COOX mit X gleich Wasserstoff und R₂ und R₃ zusammen Piperidin sind.

11. Beschichtung nach einem der vorhergehenden Ansprüche, **dadurch gekennzeichnet, daß** die Komponenten der Beschichtung in einem Lösungsmittel, vorzugsweise Chloroform, gelöst vorliegen, wobei die Anteile der einzelnen Komponenten pro Milliliter des Lösungsmittels folgende Werte betragen:
100 bis 300, vorzugsweise 150 bis 160 Milligramm Arzneistoffträger;
0,5 bis 20 Gew.-%, vorzugsweise bis 10 Gew.-% eines direkt wirkenden Antithrombins (CRC220) ;
0,3 bis 2 Gew.-%, vorzugsweise 0,5 - 1 Gew.-% eines Prostaglandinderivats oder eines entsprechenden Analogens;
0,5 bis 10 % Hirudin.

12. Beschichtung nach Anspruch 10, **dadurch gekennzeichnet, daß** die Mischung bevorzugt 5 Gew.-% jeder einzelnen Substanz aufweist.

## Claims

1. A coating for biomaterial which can be introduced into the bloodstream or into the tissue of the human body, such as infusion, cardiac or balloon catheters, electrodes for heart pacemakers and defibrillators, suture material, oxygenators, support constructions for vessels (stents) or the like, said coating preventing in particular blood coagulating on the biomaterial due to adhesion of plasmatic or cellular constituents, and the coating having a blood- and tissue-compatible medicinal substance carrier which is dissolved in an organic solvent and in which at least one medicament is incorporated and which, after application to the biomaterial and evaporation of the solvent, is permanently biodegraded in the body, **characterized in that** an inhibitor of serine proteases is present in homogeneously dispersed dissolved form in the medicinal substance carrier, said inhibitor being soluble together with the medicinal substance carrier in the same organic solvent so that application to the biomaterial and evaporation of the solvent result in a homogeneous coating composed of medicinal substance carrier and inhibitor and having a function comparable to endothelium.

2. A coating according to claim 1, **characterized in that** the inhibitor is a directly acting thrombin inhibitor.

3. A coating according to claim 2, **characterized in that** the thrombin inhibitor suppresses contact activation of blood coagulation.

4. A coating according to any of the preceding claims, **characterized in that**, to achieve an endothelium-like action, furthermore a prostaglandin or prostacyclin or a corresponding derivative is added to the solvent together with the medicinal substance carrier and the inhibitor and is contained in dissolved form in the medicinal substance carrier.

5. A coating according to any of the preceding claims, **characterized in that** a rapidly releasable antithrombin, such as hirudin, is additionally incorporated into the medicinal substance carrier.

6. A coating according to any of the preceding claims, **characterized in that** a poly-D,L-lactide is used as the medicinal substance carrier.

7. A coating according to any of the preceding claims, **characterized in that** the individual components of the coating are dissolved in chloroform.

8. A coating according to any of the preceding claims, **characterized in that** the coating forms on the biomaterial a paint-like adhesive layer with layer thicknesses of less than 100 micrometers, preferably less than 50 micrometers or 10 micrometers.

9. A coating according to any of the preceding claims, **characterized in that** a compound of the following structure is used as soluble antithrombin: 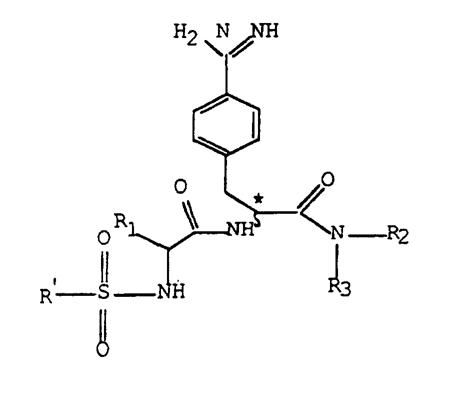 where
R' is a naphthalene ring bonded in the alpha or beta position and optionally derivatized with alkyl groups containing up to 3 carbon atoms, and/or alkoxy groups with in each case up to 3 carbon atoms, or is a tetralin ring or indan ring bonded in the alpha or beta position and optionally derivatized with alkyl groups comprising up to 3 carbon atoms, and/or else alkoxy groups with in each case up to 3 carbon atoms,
or is a phenyl ring optionally derivatized with alkyl groups containing up to 4 carbon atoms, and/or with up to three groups with the structure O-X where O is oxygen and X is hydrogen, methyl, ethyl, n-propyl, i-propyl or tert-butyl, and/or with a group with the structure -COOY where Y is hydrogen, methyl, ethyl, n-propyl, i-propyl, tert-butyl, i-butyl, i-pentyl or neo-pentyl,
or is a chroman system preferably derivatized with up to 5 alkyl groups containing up to 3 carbon atoms,
R₁ is a group with the structure A-B where A = -(CH₂)ₙ- and n = 1-4 and B is an acidic function selected from the group consisting of carboxyl function which can optionally be esterified or present as an amide, the esters containing an alcohol with up to 17 carbon atoms, sulfonic acid function, a function of a phosphorus acid, a boronic acid function and tetrazole group, or R₁ is a group with the structure A-B-C where A has the above meaning, B is carbonyl or sulfonyl, and group C is derived from an N-bonded alpha, beta, gamma or delta amino acid or the group of N-glycosidically linked uronic acids, and
R₂ and R₃ can be identical or different and are alkyl groups with up to 4 carbon atoms or together form a heterocyclic ring which has up to 8 ring members and can be derivatized with a hydroxyl group or a hydroxyalkyl group with up to 3 carbon atoms, and said hydroxyl group is optionally in esterified form, the corresponding acids being carboxylic acids containing up to 17 carbon atoms and in which the carbon atom labeled with * has the R or S structure, but preferably the R structure (CRC220).

10. A coating according to claim 9, **characterized in that** R' is 4-methoxy-2,3,6-trimethylphenyl-, R₁ is -CH2-COOX with X equal to hydrogen and R₂ and R₃ together are piperidine.

11. A coating according to any of the preceding claims, **characterized in that** the components of the coating are present dissolved in a solvent, preferably chloroform, the contents of the individual components per milliliter of solvent having the following values:
100 to 300, preferably 150 to 160, milligrams of medicinal substance carrier;
0.5 to 20% by weight, preferably up to 10% by weight, of a directly acting antithrombin (CRC220) ;
0.3 to 2% by weight, preferably 0.5-1% by weight, of a prostaglandin derivative or a corresponding analog;
0.5 to 10% hirudin.

12. A coating according to claim 10, **characterized in that** the mixture preferably comprises 5% by weight of each individual substance.

## Revendications

1. Revêtement destiné à un biomatériau pouvant être introduit dans le flux sanguin ou dans le tissu du corps humain, tel qu'un cathéter de perfusion, un cathéter cardiaque ou une sonde à ballonnet, des électrodes pour stimulateurs cardiaques et des défibrillateurs, du matériau de suture, des oxygénateurs, des ancrages vasculaires (stents) ou similaires, ce revêtement permettant en particulier d'empêcher la coagulation sanguine sur le biomatériau par l'adhérence d'éléments plasmatiques ou cellulaires, et le revêtement présentant un support de médicament toléré par le sang et les tissus, qui est dissout dans un solvant organique, et dans lequel est stocké au moins un médicament et qui, après application sur le biomatériau et après évaporation du solvant, est biodégradé de manière permanente dans le corps, **caractérisé en ce que** dans le support de médicament se trouve un inhibiteur des protéases de sérine sous forme dissoute répartie de manière homogène, cet inhibiteur étant soluble conjointement avec le support de médicament dans le même solvant organique de sorte qu'après avoir été appliqué sur le biomatériau et après l'évaporation du solvant, on obtient un revêtement homogène constitué par le support de médicament et l'inhibiteur, qui présente une fonction comparable à l'endothélium.

2. Revêtement selon la revendication 1, **caractérisé en ce que** l'inhibiteur est un inhibiteur de thrombine à effet direct.

3. Revêtement selon la revendication 2, **caractérisé en ce que** l'inhibiteur de thrombine fait cesser l'activation de contact de la coagulation sanguine.

4. Revêtement selon l'une quelconque des revendications précédentes, **caractérisé en ce que** pour obtenir l'effet similaire à l'endothélium, une prostaglandine ou une prostacycline, respectivement, ou un dérivé correspondant est ajouté, conjointement avec le support de médicament et l'inhibiteur au solvant et est contenu sous forme dissoute dans le support de médicament.

5. Revêtement selon l'une quelconque des revendications précédentes, **caractérisé en ce qu'**une antithrombine rapidement libérable, telle que de l'hirudine, est incorporée additionnellement dans le support de médicament.

6. Revêtement selon l'une quelconque des revendications précédentes, **caractérisé en ce qu'**on utilise comme support de médicament un polylactide D,L.

7. Revêtement selon l'une quelconque des revendications précédentes, **caractérisé en ce que** les composants individuels du revêtement sont dissous dans du chloroforme.

8. Revêtement selon l'une quelconque des revendications précédentes, **caractérisé en ce que** le revêtement sur le biomatériau forme une couche adhérente à la manière d'une laque avec des épaisseurs de couche inférieures à 100 micromètres, de préférence inférieures à 50 micromètres ou à 10 micromètres.

9. Revêtement selon l'une quelconque des revendications précédentes, **caractérisé en ce qu'**un composé avec la structure suivante est utilisé comme antithrombine soluble : 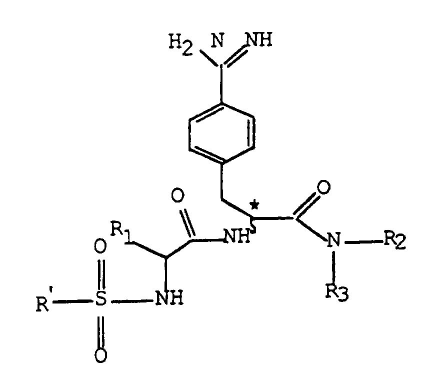 dans lequel
R' est un cycle de naphtalène lié en position alpha ou bêta, qui est dérivé, le cas échéant, avec des groupes alkyles ayant jusqu'à trois atomes de carbone et/ou des groupes alkoxy ayant chacun jusqu'à trois atomes de carbone, ou un cycle de tétraline lié en position alpha ou en position bêta ou un cycle d'indane qui est le cas échéant dérivé avec des groupes alcyles qui sont formés par jusqu'à trois atomes de carbone, et/ou aussi des groupes alkoxy ayant chacun jusqu'à trois atomes de carbone,
ou un cycle de phényle qui est dérivé, le cas échéant, avec des groupes alkyles ayant jusqu'à quatre atomes de carbone, et/ou jusqu'à trois groupes de la structure O-X, dans laquelle O est de l'oxygène et X de l'hydrogène, du méthyle, de l'éthyle, du n-propyle, du i-propyle ou du tert-butyle, et/ou avec un groupe de la structure -COOY, dans laquelle Y est de l'hydrogène, du méthyle, de l'éthyle, du n-propyle, du i-propyle ou du tert-butyle, du 1-butyle, du i-pentyle ou du néopentyle,
ou est un système chromanique qui est dérivé de préférence avec jusqu'à cinq groupes alkyles ayant jusqu'à trois atomes de carbone
R₁ est un groupe de la structure A-B, A étant égal à - (CH₂)ₙ - et n étant égal à 1-4, et B étant une fonction acide choisie parmi le groupe de fonction carboxyle qui peut, le cas échéant, être estérifié ou être sous forme d'amide, les esters ayant un alcool ayant jusqu'à 17 atomes de carbone, une fonction d'acide sulfonique, une fonction d'un acide de phosphore, une fonction d'acide de boronique et un groupe tétrazole, ou bien R1 est un groupe de la structure A-B-C, A ayant la signification précitée, B étant du carbonyle ou du sulfonyle, et le groupe C étant dérivé d'un acide aminé alpha, bêta, gamma ou delta lié à N, ou du groupe des acides uroniques à liaison glycosidique et,
R₂ et R₃ peuvent être identiques ou différents et sont des groupes alkyles avec jusqu'à quatre atomes de carbone ou forment ensemble un composé hétérocyclique ayant jusqu'à 8 termes cycliques qui peut être dérivés avec un groupe hydroxy ou un groupe hydroxyalkyle ayant jusqu'à trois atomes de carbone, et ce groupe hydroxy pouvant, le cas échéant, être sous forme estérifiée, les acides correspondants étant des acides carboxyliques qui contiennent jusqu'à 17 atomes de carbone, et dans lequel l'atome de carbone désigné par * est en configuration R ou S, mais de préférence en configuration R (CRC220).

10. Revêtement selon la revendication 9, **caractérisé en ce que** R' est du 4-methoxy-2,3,6-trimethylphenyl-, R₁ est -CH2-COOX, X étant égal à l'hydrogène, et R₂ et R₃ sont ensemble de la pipéridine.

11. Revêtement selon l'une quelconque des revendications précédentes, **caractérisé en ce que** les composants du revêtement sont dissous dans un solvant, de préférence du chloroforme, les parts des composants individuels par millilitre du solvant ont les valeurs suivantes :
100 à 300, de préférence 150 à 160 milligrammes de support de médicament ;
0,5 à 20 % en poids, de préférence jusqu'à 10 % en poids d'une antithrombine à effet direct (CRC220) ;
0,3 à 2 % en poids, de préférence 0,5 à 1 % en poids d'un dérivé de prostaglandine ou d'un analogue correspondant ;
0,5 à 10 % d'hirudine.

12. Revêtement selon la revendication 10, **caractérisé en ce que** le mélange présente de préférence 5 % en poids de chacune des substances.
